# FASCICULE DE BREVET EUROPEEN

(11) **EP 0 625 146 B1**
(45) Date de publication et mention de la délivrance du brevet: **18.12.1996**
(21) Numéro de dépôt: 93904151.3
(22) Date de dépôt: 04.02.1993
(51) Int. Cl.: C07D 305/04, C07C 271/22, C07D 263/04, A61K 31/335

(54) **NOUVEAUX ANHYDRIDES D'ACIDES, LEUR PREPARATION ET LEUR EMPLOI**
SÄUREANHYDRIDE, IHRE HERSTELLUNG UND IHRE VERWENDUNG
NOVEL ACID ANHYDRIDES AND PREPARATION AND USE THEREOF

(30) Priorité: 07.02.1992 FR 9201380
(43) Date de publication de la demande: 23.11.1994
(73) Titulaire: RHONE-POULENC RORER S.A., 92160 Antony (FR)
(72) Inventeur: MAS, Jean-Manuel, F-69100 Villeurbanne (FR)
(74) Mandataire: Pilard, Jacques
(86) Numéro de dépôt international: FR9300111
(87) Numéro de publication internationale: WO9316058

(56) Documents cités:
- EP-A- 0 336 840
- EP-A- 0 336 841
- EP-A- 0 400 971

## Description

La présente invention concerne de nouveaux anhydrides de formule générale : 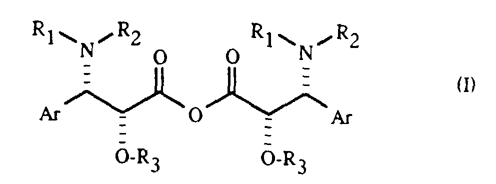 leur préparation et leur emploi.

Dans la formule générale (I),
- Ar représente un radical aryle, et
- ou bien R₁ représente un radical benzoyle ou t.butoxycarbonyle, R₂ représente un atome d'hydrogène et R₃ représente un groupement protecteur de la fonction hydroxy,
- ou bien R₁ représente un radical t.butoxycarbonyle et R₂ et R₃ forment ensemble un hétérocycle saturé à 5 ou 6 chaînons.

Plus particulièrement, Ar représente un radical phényle ou α- ou β-naphtyle éventuellement substitué, les substituants pouvant être choisis parmi les atomes d'halogène (fluor, chlore, brome, iode) et les radicaux alcoyles, aryles, arylalcoyles, alcoxy, alcoylthio, aryloxy, arylthio, hydroxy, mercapto, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle étant entendu que les radicaux alcoyles et les portions alcoyles des autres radicaux contiennent 1 à 4 atomes de carbone et les radicaux aryles sont des radicaux phényles ou α- ou β-naphtyles.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (t.butoxycarbonyiaminol.

Plus particulièrement, R₃ représente un groupement protecteur de la fonction hydroxy choisi parmi les radicaux méthoxyméthyle, éthoxy-1 éthyle, benzyloxyméthyle, (β-triméthylsilyléthoxyl méthyle, tétrahydropyrannyle, trichloro-2,2,2 éthoxyméthyle ou trichloro-2,2,2 éthoxycarbonyle.

Plus particulièrement, lorsque R₂ et R₃ forment ensemble un hétérocycle saturé à 5 ou 6 chaînons, celui-ci représente un cycle oxazolidine éventuellement gem-disubstitué en position -2.

Selon la présente invention, les nouveaux anhydrides de formule générale (I) peuvent être obtenus par action d'un agent de déshydratation tel qu'un imide comme le dicyclohexylcarbodiimide sur l'acide de formule générale : 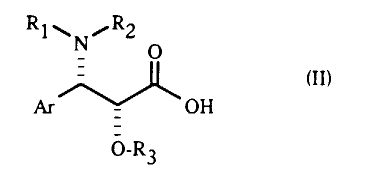 dans laquelle Ar, R₁, R₂ et R₃ sont définis comme précédemment.

Généralement, on utilise de 0,5 à 1 mole d'agent de déshydratation par mole d'acide mise en oeuvre.

Généralement, la réaction est effectuée dans un solvant organique choisi parmi les hydrocarbures aliphatiques halogénés tels que le dichlorométhane ou le chloroforme et les hydrocarbures aromatiques tels que le benzène, le toluène ou les xylènes.

Généralement la réaction est mise en oeuvre à une température comprise entre 0 et 30°C.

L'anhydride obtenu peut être séparé du mélange réactionnel selon les techniques habituelles. Cependant, il peut être particulièrement avantageux d'utiliser l'anhydride obtenu préparé extemporanément sans isolement préalable à son emploi en particulier dans des réactions d'estérification.

Les anhydrides de formule générale (I) sont généralement plus stables que les acides dont ils dérivent dans les réactions d'estérification et ils peuvent conduire à des réactions plus facilement reproductibles.

Les nouveaux anhydrides de formule générale (I) sont particulièrement utiles pour préparer le taxol ou le taxotère ou leurs dérivés de formule générale : 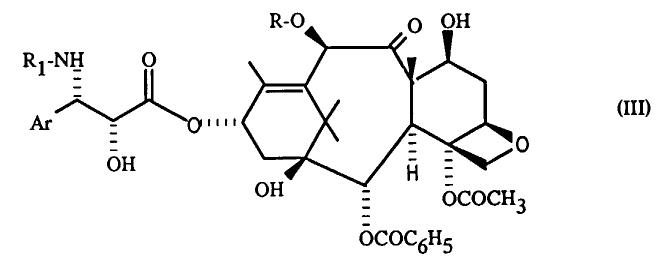 dans laquelle R représente un atome d'hydrogène ou un radical acétyle et R₁ et Ar sont définis comme précédemment, qui présentent des propriétés antitumorales particulièrement intéressantes.

Il est connu de préparer les produits de formule générale (III) à partir d'un acide et d'un dérivé de la baccatine III ou de la désacétyl-10 baccatine III convenablement protégée dans les conditions décrites dans EP-A-0 336 840 et EP-A-0 336 841.

Selon l'invention, les produits de formule générale (III) peuvent être obtenus :
- soit par action d'un anhydride de formule générale (I) dans laquelle Ar est défini comme précédemment, R₁ représente un radical benzoyle ou t.butoxycarbonyle, R₂ représente un atome d'hydrogène et R₃ représente un groupement protecteur de la fonction hydroxy, sur un dérivé de la baccatine III ou de la désacétyl-10 baccatine III de formule générale : 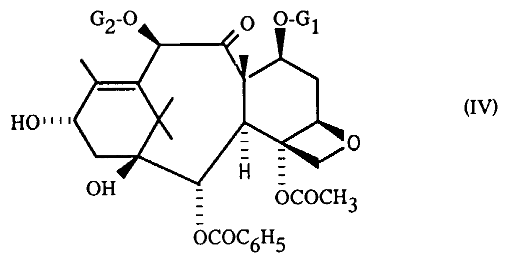
dans laquelle G₁ représente un groupement protecteur de la fonction hydroxy tel qu'un radical trichloro-2,2,2 éthoxycarbonyle ou trialcoylsilyle dont chaque partie alcoyle contient 1 à 4 atomes de carbone et G₂ représente un radical acétyle ou un groupement protecteur de la fonction hydroxy tel qu'un radical trichloro-2,2,2 éthoxycarbonyle, pour obtenir un produit de formule générale : 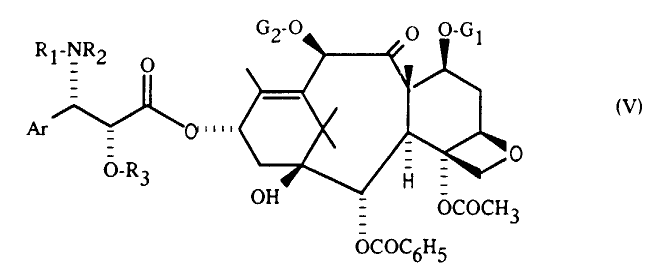 dans laquelle Ar, R₁, R₂, R₃, G₁ et G₂ sont définis comme ci-dessus, suivi du remplacement des radicaux G₁, R₃ et éventuellement G₂ par des atomes d'hydrogène pour obtenir le produit de formule générale (III).

L'estérification de l'alcool de formule générale (IV) est généralement effectuée en présence d'un agent d'activation tel qu'une aminopyridine comme la diméthylamino-4 pyridine en opérant dans un solvant organique tel que le benzène, le toluène, les xylènes, l'éthylbenzène, l'isopropylbenzène ou le chlorobenzène à une température comprise entre 0 et 90°C.

Généralement, on utilise de 0,6 à 1,6 mole d'anhydride de formule générale (I) par mole d'alcool de formule générale (IV).

Généralement, on utilise de 0,1 à 1 mole d'agent d'activation par mole d'alcool de formule générale (IV).

Il est particulièrement avantageux d'opérer dans un milieu dans lequel la concentration en alcool de formule générale (IV) dans le solvant est comprise entre 1 et 30 % (poids/volume).

Selon la nature des groupements protecteurs G₁, G₂ et R₃, leur remplacement par des atomes d'hydrogène peut être effectué au moyen de zinc en présence d'acide acétique ou d'un acide minéral ou organique tel que l'acide chlorhydrique ou l'acide acétique en solution dans un alcool aliphatique contenant 1 à 3 atomes de carbone en présence de zinc lorsque les groupements protecteurs représentent au moins un radical trichloro-2,2,2 éthoxycarbonyle, soit au moyen d'un acide tel que l'acide chlorhydrique dans un alcool aliphatique contenant 1 à 3 atomes de carbone à une température voisine de 0°C lorsque les groupements protecteurs représentent au moins un radical trialcoylsilyle.

Lorsque le groupement protecteur R₃ représente un radical méthoxyméthyle, éthoxy-1 éthyle, benzyloxyméthyle, (β-triméthylsilyléthoxy) méthyle ou tétrahydropyrannyle, il est possible de remplacer ce groupement protecteur par un atome d'hydrogène par traitement en milieu acide à une température comprise entre 0 et 30°C pour obtenir un produit de formule générale : 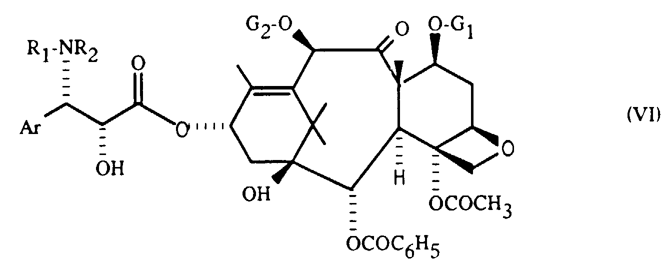 qui peut être purifié préalablement au remplacement des groupements protecteurs G₁ et G₂ par des atomes d'hydrogène dans les conditions décrites précédemment.
- soit par action d'un anhydride de formule générale (I) dans laquelle Ar est défini comme précédemment, R₁ représente un radical t.butoxycarbonyle et R₂ et R₃ forment ensemble un hétérocycle saturé à 5 ou 6 chaînons sur un produit de formule générale (IV), pour obtenir un produit de formule générale (V) dans laquelle Ar est défini comme précédemment, R₁ représente un radical t.butoxycarbonyle et R₂ et R₃ forment ensemble un hétérocycle saturé à 5 ou 6 chaînons, qui est traité par un acide minéral ou organique éventuellement dans un alcool dans des conditions qui sont sans effet sur les groupements protecteurs G₁ et G₂ de façon à obtenir un produit de formule générale : 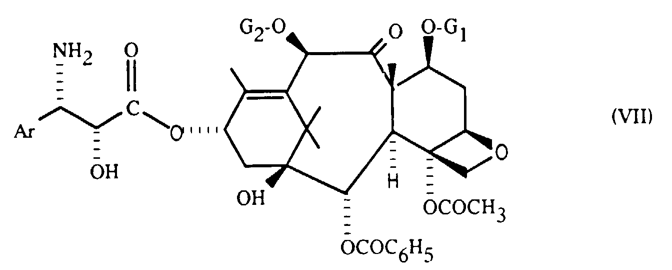
dans laquelle Ar est défini comme précédemment, G1 représente un groupement protecteur de la fonction hydroxy, de préférence un radical trichloro-2,2,2 éthoxycarbonyle et G₂ représente un radical acétyle ou un groupement protecteur de la fonction hydroxy tel que le radical trichloro-2,2,2 éthoxycarbonyle, qui est traité par un composé qui permet d'introduire, sur la fonction amino, un radical benzoyle ou t.butoxycarbonyle pour obtenir un produit de formule générale (VI) dans laquelle Ar, G₁ et G₂ sont définis comme ci-dessus dont on remplace les groupements protecteurs G₁ et G₂ par des atomes d'hydrogène dans les conditions décrites précédemment.

Généralement, l'estérification du produit de formule générale (IV) est effectuée en présence d'un agent d'activation tel qu'une aminopyridine comme la diméthylamino-4 pyridine en opérant dans un solvant organique tel que le benzène, le toluène, les xylènes, l'éthylbenzène, l'isopropylbenzène ou le chlorobenzène à une température comprise entre0 et 90°C.

Généralement, on utilise de 0,6 à 1,6 mole d'anhydride de formule générale (I) par mole d'alcool de formule générale (IV).

Généralement, on utilise de 0,1 à 1 mole d'agent d'activation par mole d'alcool de formule générale (IV).

Il est particulièrement avantageux d'opérer dans un milieu dans lequel la concentration en alcool de formule générale (IV) est comprise entre 1 et 30 % (poids/volume).

Généralement, le produit de formule générale (VII) est obtenu en traitant le produit de formule générale (V) dans laquelle Ar est défini comme précédemment, R₁ représente un radical t.butoxycarbonyle et R₂ et R₃ forment ensemble un hétérocycle saturé à 5 ou 6 chaînons, par l'acide formique éventuellement dans un alcool tel que l'éthanol ou par l'acide chlorhydrique gazeux dans un alcool tel que l'éthanol.

L'introduction d'un groupement benzoyle ou t.butoxycarbonyle est effectuée en faisant réagir le chlorure de benzoyle ou le dicarbonate de di-tert.butyle sur le produit de formule générale (VII) en opérant dans un solvant organique tel que le chlorure de méthylène en présence d'une base minérale telle que le bicarbonate de sodium ou d'une base organique telle qu'une amine tertiaire comme la triéthylamine.

Les produits de formule générale (III) obtenus par la mise en oeuvre du procédé selon l'invention peuvent être purifiés selon les méthodes habituelles.

Les exemples suivants illustrent la présente invention.

### EXEMPLE 1

A une solution de 1,72 g d'acide phényl-3 t.butoxycarbonylamino-3 (éthoxy-1 éthoxy)-2 propionique-(2R,3S) (4,87 mmoles) dans 4 cm3 de chlorure de méthylène anhydre, on ajoute, à -10°C et sous atmosphère d'argon, 0,206 g de dicyclohexylcarbodiimide en solution dans 1 cm3 de chlorure de méthylène anhydre.

Le mélange réactionnel est agité pendant 40 minutes en laissant la température remonter au voisinage de 20°C.

La dicyclohexylurée formée est séparée par filtration sous atmosphère inerte et le filtrat est concentré à sec sous pression réduite (20 mm de mercure ; 2,7 kPa) à 30°C.

On obtient ainsi 1,72 g d'anhydride de l'acide phényl-3 t.butoxycarbonylamino-3 (éthoxy-1 éthoxy)-2 propionique-(2R,3S) dont les caractéristiques sont les suivantes :
- point de fusion : 43°C
- spectre infra-rouge (nujol) : bandes d'absorption caractéristiques à 3450-3330, 1835, 1764 et 1722 cm⁻¹
- spectre de résonance magnétique nucléaire du proton (mélange des trois isomères) (360 MHz, CDCl₃/HMDS, déplacements chimiques en ppm, T = 40°C) :
- isomère A : 0,93 (6H, t) ; 0,99 (6H, d) ; 1,37 (18H, s élargi) ; 3,27 (4H, multiplet) ; 4,36 (2H, q) ; 4,44 (2H, s élargi) ; 5,53 (2H, s élargi) ; 7,11 (4H, d) ; 7,20 (2H, t) ; 7,29 (4H, t)
- isomère B : 0,93 (6H, t) ; 9,99 (6H, d) ; 1,37 (18H, s élargi) ; 3,27 (4H, multiplet) ; 4,37 (2H, q) ; 4,44 (2H, s élargi) ; 5,53 (2H, s élargi) ; 7,11 (4H, d) ; 7,20 (2H, t) ; 7,29 (4H, t)
- isomère C : 0,73 (6H, t) ; 1,12 (3H, d) ; 1,13 (3H, d) 1,37 (18H, s élargi) ; 2,61 (2H, m) ; 3,08 (2H, m) ; 4,58 (2H, s élargi) ; 4,72 (1H, q) ; 4,73 (1H, q) ; 5,53 (2H, s élargi) ; 7,11 (4H, d) ; 7,20 (2H, t) ; 7,29 (4H, t).

### EXEMPLE 2

Dans un réacteur de 250 cm3, on introduit 22,16 g d'acide phényl-3 t.butoxycarbonylamino-3 (éthoxy-1 éthoxy)-2 propionique-(2R,3S) (6,28 x 10⁻² mole) et 12,43 g de dicyclohexylcarbodiimide (6,02 x 10⁻² mole) dans 85 cm3 de toluène sec. On agite pendant 30 minutes.

Après filtration de la dicyclohexylurée formée, la solution obtenue est ajoutée, en 8 heures, à une solution de 21 g d'acétoxy-4 benzoyloxy-2α époxy-5β,20 dihydroxy-1,13α oxo-9 bis-(trichloro-2,2,2 éthoxy) carbonyloxy-7β,10β taxène-11 titrant 95 % (2,24 x 10⁻² mole) et de 0,61 g de diméthylamino-4 pyridine dans 84 cm3 de toluène sec à 75°C.

On agite encore pendant 2 heures après la fin de l'addition. Après refroidissement à une température voisine de 20°C, la dicyclohexylurée est séparée par filtration. Le filtrat est concentré à sec et le résidu est repris par 150 cm3 de cyclohexane. Après solubilisation totale à 60°C, la solution est versée sur 350 cm3 d'heptane refroidi à une température comprise entre 1 et 5°C. Le précipité formé est séparé par filtration, lavé à l'heptane froid puis séché sous pression réduite. On obtient ainsi 38 g d'un produit légèrement brun dont l'analyse par chromatographie liquide à haute performance (CLHP) montre qu'il contient 25,5 g de tert.butoxycarbonylamino-3 phényl-3 (éthoxy-1 éthoxy)-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α contenant 15 % d'épimère 2S,3S.

Le produit obtenu, traité dans les conditions décrites dans le brevet américain US 4 924 011, fournit le tert-butoxycarbonylamino-3 phényl-3 hydroxy-2 propionate-(2R,3S) d'acétoxy-4 benzoyloxy-2α époxy-5β,20 trihydroxy-1,7β,10β oxo-9 taxène-11 yle-13α.

### EXEMPLE 3

A une solution de 1,6 g d'acide t.butoxycarbonyl-3 diméthyl-2,2 phényl-4 oxazolidinecarboxylique-5-(4S,5R) dans 5 cm3 de chlorure de méthylène anhydre, on ajoute, à une température voisine de 20°C et sous atmosphère d'argon, 0,206 g de dicyclohexylcarbodiimide.

Le mélange réactionnel est agité pendant 35 minutes.

La dicyclohexylurée formée est séparée par filtration et le filtrat est concentré à sec sous pression réduite (20 mm de mercure ; 2,7 kPa) à 30°C.

On obtient ainsi 1,5 g d'anhydride de l'acide t.butoxycarbonyl-3 diméthyl-2,2 phényl-4 oxazolidinecarboxylique-5-(4S,5R) dont les caractéristiques sont les suivantes :
- point de fusion : 46°C
- spectre infra-rouge (nujol) : principales bandes d'absorption caractéristiques à 1836, 1764 et 1703 cm⁻¹
- spectre de résonance magnétique nucléaire du proton (360 MHz ; DMSO/HMDS ; déplacements chimiques en ppm) : 1,15 (s élargi, 9H) ; 1,57 (s, 3H) ; 1,64 (s, 3H) ; 4,52 (d, 1H); 5,03 (s élargi, 1H) ; 7,28 (m, 5H).

### EXEMPLE 4

En opérant comme dans l'exemple 2 mais en utilisant l'anhydride d'un acide tert-butoxycarbonyl-3 diméthyl-2,2 aryl-4 oxazolidinecarboxylique-5-(4S,5R) préparé dans les conditions de l'exemple 3 et en passant intermédiairement par le produit de formule générale (VII) sur lequel on fait réagir le dicarbonate de di-tert.butyle ou le chlorure de benzoyle, sont préparés les produits suivants :
- tert-butoxycarbonylamino-3 (méthyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dont le pouvoir rotatoire est [α]²⁰ _{D} = -32° (c = 0,1; méthanol),
- tert-butoxycarbonylamino-3 (fluoro-3 phényl)-3 hydroxy-2 propionate-(2R,3S) d'acétoxy-4 benzoyloxy-2α époxy-5β,20 trihydroxy-1,7β,10β oxo-9 taxène-11 yle13α dont le pouvoir rotatoire est [α]²⁰ _{D} = -34° (c = 0,59 ; méthanol),
- tert-butoxycarbonylamino-3 (fluoro-2 phényl)-3 hydroxy-2 propionate-(2R,3S) d'acétoxy-4 benzoyloxy-2α époxy-5β,20 trihydroxy-1,7β,10β oxo-9 taxène-11 yle-13α dont le pouvoir rotatoire est [α]²⁰ _{D} = -42° (c = 0,58 ; méthanol),
- tert-butoxycarbonylamino-3 (chloro-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dont le pouvoir rotatoire est [α]²⁰ _{D} = -27° (c = 0,97 ; méthanol),
- tert-butoxycarbonylamino-3 (méthoxy-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dont le pouvoir rotatoire est [α]²⁰ _{D} = -32° (c = 0,47 ; méthanol),
- tert-butoxycarbonylamino-3 (fluoro-4 phényl)-3 hydroxy-2 propionate-(2R ,3S) d'acétoxy-4 benzoyloxy-2α époxy-5β,20 trihydroxy-1,7β,10β oxo-9 taxène-11 yle-13α dont le pouvoir rotatoire est [α]²⁰ _{D} = -35° (c = 0,49 ; méthanol) et le
- benzoylamino-3 hydroxy-2 phényl-3 propionate-(2R,3S) de diacétoxy-4,10β benzoyloxy-2α époxy-5β,20 dihydroxy-1,7β oxo-9 taxène-11 yle-13α (ou taxol).

La présente invention concerne également les produits de formule générale (III) lorsqu'ils sont obtenus par un procédé mettant en oeuvre un anhydride de formule générale (I).

La présente invention concerne également les compositions antitumorales qui contiennent un produit de formule générale (III) lorsqu'il est obtenu par un procédé mettant en oeuvre un anhydride de formule générale (I).

## Revendications

1. Nouveaux anhydrides de formule générale : 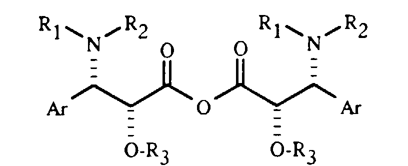 dans laquelle Ar représente un radical aryle et, ou bien R₁ représente un radical benzoyle ou t.butoxycarbonyle, R₂ représente un atome d'hydrogène et R₃ représente un groupement protecteur de la fonction hydroxy, ou bien R₁ représente un radical t.butoxycarbonyle et R₂ et R₃ forment ensemble un hétérocycle saturé à 5 ou 6 chaînons.

2. Nouveaux anhydrides selon la revendication 1 caractérisés en ce que Ar représente un radical phényle ou α- ou β-naphtyle éventuellement substitué, les substituants étant choisis parmi les atomes d'halogène et les radicaux alcoyles, aryles, arylalcoyles, alcoxy, alcoylthio, aryloxy, arylthio, hydroxy, mercapto, acylamino, alcoxycarbonylamino, amino, alcoylamino, dialcoylamino, carboxy, alcoxycarbonyle, carbamoyle, dialcoylcarbamoyle, cyano, nitro et trifluorométhyle étant entendu que les radicaux alcoyles et les portions alcoyles des autres radicaux contiennent 1 à 4 atomes de carbone et que les radicaux aryles sont des radicaux phényles ou α- ou β-naphtyles.

3. Nouveaux anhydrides selon la revendication 1 caractérisés en ce que Ar représente un radical phényle éventuellement substitué par un atome de chlore ou de fluor, ou par un radical alcoyle contenant 1 à 4 atomes de carbone, alcoxy contenant 1 à 4 atomes de carbone, dialcoylamino dont chaque partie alcoyle contient 1 à 4 atomes de carbone, acylamino contenant 1 à 4 atomes de carbone ou alcoxycarbonylamino contenant 1 à 4 atomes de carbone.

4. Nouveaux anhydrides selon l'une des revendications 1, 2 ou 3 caractérisés en ce que, lorsque R₂ représente un atome d'hydrogène, R₃ représente un radical méthoxyméthyle, éthoxy-1 éthyle, benzyloxyméthyle, (β-triméthylsilyléthoxy) méthyle, tétrahydropyrannyle, trichloro-2,2,2 éthoxyméthyle ou trichloro-2,2,2 éthoxycarbonyle.

5. Nouveaux anhydrides selon l'une des revendications 1, 2 ou 3 caractérisés en ce que R₂ et R₃ forment ensemble un cycle oxazolidine éventuellement gem-disubstitué en position -2.

6. Procédé de préparation d'un anhydride selon l'une des revendications 1, 2, 3, 4 ou 5 caractérisé en ce que l'on fait réagir un agent de déshydratation sur un acide de formule générale : 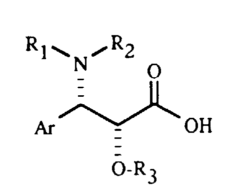 dans laquelle Ar, R₁, R₂ et R₃ sont définis comme dans les revendications 1, 2, 3, 4 ou 5.

7. Procédé selon la revendication 6 caractérisé en ce que l'agent de déshydratation est le dicyclohexylcarbodiimide.

8. Procédé selon l'une des revendications 6 ou 7 caractérisé en ce que l'on opère dans un solvant organique choisi parmi les hydrocarbures aliphatiques halogénés et les hydrocarbures aromatiques.

9. Procédé selon l'une des revendications 6, 7 ou 8 caractérisé en ce que l'on opère à une température comprise entre 0 et 30°C.

10. Utilisation d'un anhydride selon l'une des revendications 1, 2, 3 ou 4 éventuellement préparé in situ pour la préparation d'un produit de formule générale : 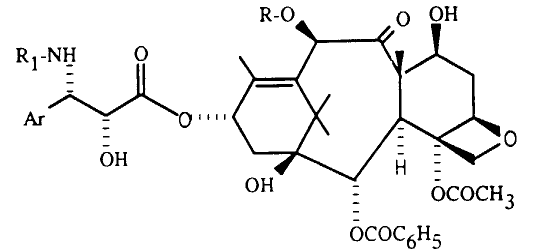 dans laquelle R représente un atome d'hydrogène ou un radical acétyle, R₁ représente un radical benzoyle ou tert-butoxycarbonyle et Ar est défini comme dans l'une des revendications 1, 2 ou 3 caractérisé en ce que l'on fait réagir un anhydride selon l'une des revendications 1, 2, 3 ou 4 pour lequel Ar est défini comme dans l'une des revendications 1, 2 ou 3, R₁ représente un radical benzoyle ou tert-butoxycarbonyle, R₂ représente un atome d'hydrogène et R₃ représente un groupement protecteur de la fonction hydroxy défini comme dans les revendications 1 ou 4, sur un dérivé de la baccatine III ou de la désacétyl-10 baccatine III, de formule générale : 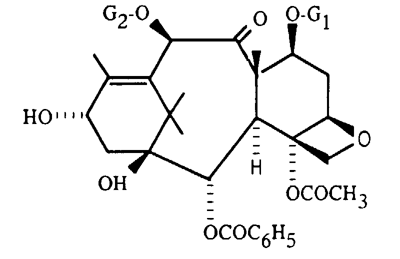 dans laquelle G₁ représente un groupement protecteur de la fonction hydroxy et G₂ représente un radical acétyle ou un groupement protecteur de la fonction hydroxy, pour obtenir un produit de formule générale : 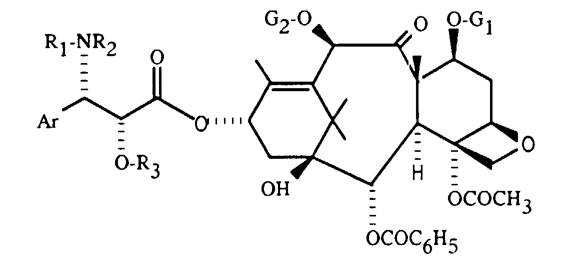 dans laquelle Ar, R₁, R₂, R₃, G₁ et G₂ sont définis comme ci-dessus, puis remplace les groupements protecteurs R₃, G₁ et G₂ par un atome d'hydrogène par application des méthodes connues.

11. Utilisation d'un anhydride selon l'une des revendications 1, 2, 3 ou 5 éventuellement préparé in situ pour la préparation d'un produit de formule générale : 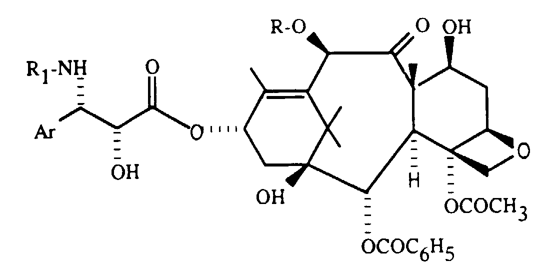 dans laquelle R représente un atome d'hydrogène ou un radical acétyle, R₁ représente un radical tert-butoxycarbonyle et Ar est défini comme dans l'une des revendications 1, 2 ou 3 caractérisé en ce que l'on fait réagir un anhydride selon l'une des revendications 1, 2, 3 ou 5 pour lequel Ar est défini comme dans l'une des revendications 1, 2 ou 3, R₁ représente un radical tert-butoxycarbonyle et R₂ et R₃ forment ensemble un hétérocycle saturé à 5 ou 6 chaînons défini comme dans l'une des revendications 1 ou 5, sur un dérivé de la baccatine III ou de la désacétyl-10 baccatine III de formule générale : 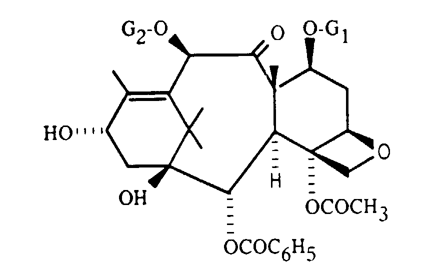 dans laquelle G₁ représente un groupement protecteur de la fonction hydroxy et G₂ représente un radical acétyle ou un groupement protecteur de la fonction hydroxy, pour obtenir un produit de formule générale : 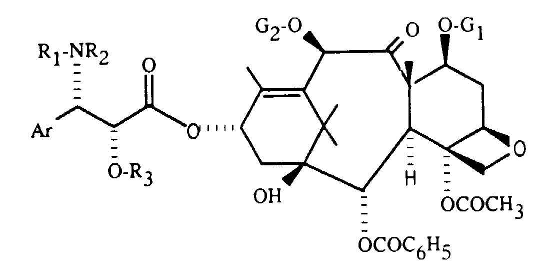 dans laquelle R₁ représente un radical tert-butoxycarbonyle, R₂ et R₃ forment ensemble un hétérocycle saturé à 5 ou 6 chaînons et Ar est défini comme dans l'une des revendications 1, 2 ou 3, G₁ représente un groupement protecteur de la fonction hydroxy et G₂ représente un radical acétyle ou un groupement protecteur de la fonction hydroxy, que l'on traite en milieu acide dans des conditions qui sont sans effet sur les groupements protecteurs G₁ et G₂ pour obtenir un produit de formule générale : 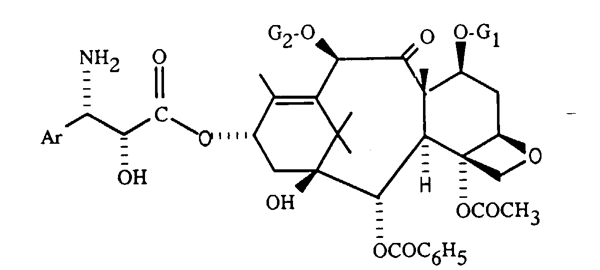 dans laquelle Ar, G₁ et G₂ sont définis comme ci-dessus, que l'on traite par un composé qui permet d'introduire un radical benzoyle ou tert-butoxycarbonyle sur la fonction amino, puis remplace les groupements protecteurs G₁ et G₂ par un atome d'hydrogène selon les méthodes connues.

## Patentansprüche

1. Neue Anhydride der allgemeinen Formel 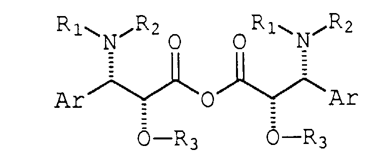 in der Ar einen Arylrest darstellt, und entweder
R₁ einen Rest Benzoyl oder tert.-Butoxycarbonyl bedeutet, R₂ ein Wasserstoffatom ist und R₃ eine Schutzgruppe für die Hydroxyfunktion darstellt, oder
R₁ einen Rest tert.-Butoxycarbonyl darstellt und R₂ und R₃ zusammen einen gesättigten Heterocyclus mit 5 oder 6 Ringgliedern bilden.

2. Neue Anhydride nach Anspruch 1, dadurch gekennzeichnet, daß Ar einen gegebenenfalls substituierten Rest Phenyl oder α- oder β-Naphthyl darstellt, wobei die Substituenten ausgewählt werden unter den Halogenatomen und den Resten Alkyl, Aryl, Arylalkyl, Alkoxy, Alkylthio, Aryloxy, Arylthio, Hydroxy, Mercapto, Acylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Alkylreste und Alkylteile der anderen Reste 1 bis 4 Kohlenstoffatome enthalten und die Arylreste Phenylreste oder α- oder β-Naphthylreste sind.

3. Neue Anhydride nach Anspruch 1, dadurch gekennzeichnet, daß Ar einen Phenylrest darstellt, gegebenenfalls substituiert durch ein Chloratom oder Fluoratom oder durch einen Alkylrest mit 1 bis 4 Kohlenstoffatomen, einen Alkoxyrest mit 1 bis 4 Kohlenstoffatomen, einen Dialkylaminorest, dessen Alkylteil 1 bis 4 Kohlenstoffatome enthält, einen Acylaminorest mit 1 bis 4 Kohlenstoffatomen oder einen Alkoxycarbonylaminorest mit 1 bis 4 Kohlenstoffatomen.

4. Neue Anhydride nach einem der Ansprüche 1, 2 oder 3, dadurch gekennzeichnet, daß in dem Fall, wo R₂ ein Wasserstoffatom ist, R₃ einen Rest Methoxymethyl, 1-Ethoxyethyl, Benzyloxymethyl, (β-Trimethylsilylethoxy)-methyl, Tetrahydropyranyl, 2,2,2-Trichlor-ethoxymethyl oder 2,2,2-Trichlor-ethoxycarbonyl darstellt.

5. Neue Anhydride nach einem der Ansprüche 1, 2 oder 3, dadurch gekennzeichnet, daß R₂ und R₃ zusammen einen Oxazolidinring bilden, gegebenenfalls geminal disubstituiert in Position 2.

6. Verfahren zur Herstellung eines Anhydrides nach einem der Ansprüche 1, 2, 3, 4 oder 5, dadurch gekennzeichnet, daß man ein Dehydratisierungsmittel mit einer Säure der allgemeinen Formel 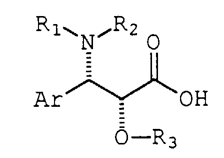 zur Reaktion bringt, in der Ar, R₁, R₂ und R₃ wie in den Ansprüchen 1, 2, 3, 4 oder 5 definiert sind.

7. Verfahren nach Anspruch 6, dadurch gekennzeichnet, daß das Dehydratisierungsmittel Dicyclohexylcarbodiimid ist.

8. Verfahren nach einem der Ansprüche 6 oder 7, dadurch gekennzeichnet, daß man in einem organischen Lösungsmittel arbeitet, ausgewählt unter den halogenierten aliphatischen Kohlenwasserstoffen und den aromatischen Kohlenwasserstoffen.

9. Verfahren nach einem der Ansprüche 6, 7 oder 8, dadurch gekennzeichnet, daß man bei einer Temperatur zwischen 0 °C und 30 °C arbeitet.

10. Verwendung eines Anhydrides nach einem der Ansprüche 1, 2, 3 oder 4, gegebenenfalls in situ hergestellt, zur Herstellung eines Produktes der allgemeinen Formel 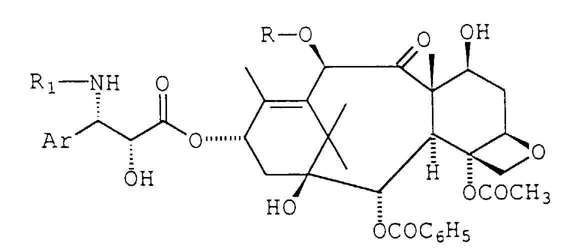 in der
R ein Wasserstoffatom oder einen Acetylrest darstellt, R₁ einen Benzoylrest oder einen Rest tert.-Butoxycarbonyl bedeutet und Ar wie in einem der Ansprüche 1, 2 oder 3 definiert ist, dadurch gekennzeichnet, daß man ein Anhydrid nach einem der Ansprüche 1, 2, 3 oder 4, worin Ar wie in einem der Ansprüche 1, 2 oder 3 definiert ist, R₁ einen Benzoylrest oder einen Rest tert.-Butoxycarbonyl bedeutet, R₂ ein Wasserstoffatom ist und R₃ eine wie in den Ansprüchen 1 oder 4 definierte Schutzgruppe für die Hydroxyfunktion darstellt, mit einem Derivat von Baccatin III oder von 10-Desacetyl-Baccatin III der allgemeinen Formel 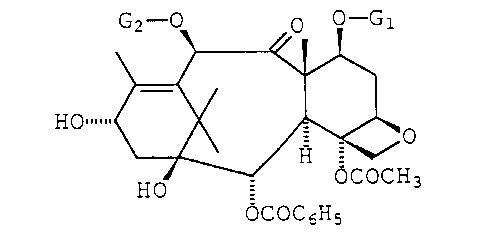 zur Reaktion bringt, in der G₁ eine Schutzgruppe für die Hydroxyfunktion darstellt und G₂ einen Acetylrest oder eine Schutzgruppe für die Hydroxyfunktion bedeutet, um ein Produkt der allgemeinen Formel 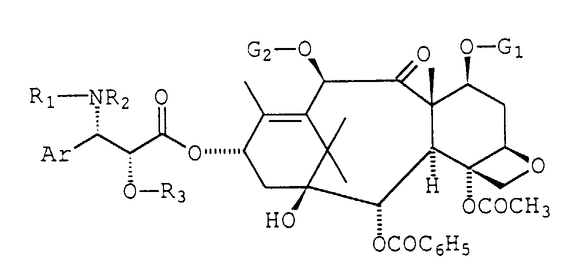 zu erhalten, worin Ar, R₁, R₂, R₃, G₁ und G₂ wie oben definiert sind, und anschließend die Schutzgruppen R₃, G₁ und G₂ mit Hilfe von bekannten Methoden durch Wasserstoffatome ersetzt.

11. Verwendung eines Anhydrides nach einem der Ansprüche 1, 2, 3 oder 5, gegebenenfalls in situ hergestellt, zur Herstellung eines Produktes der allgemeinen Formel 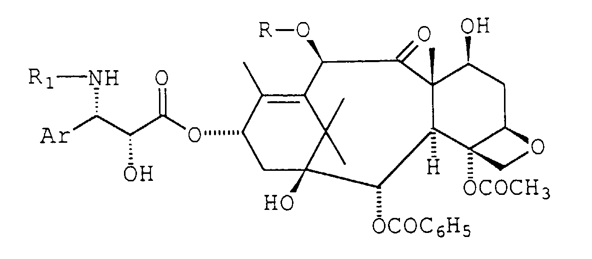 in der
R ein Wasserstoffatom oder einen Acetylrest darstellt, R₁ einen Rest tert.-Butoxycarbonyl bedeutet und Ar wie in einem der Ansprüche 1, 2 oder 3 definiert ist, dadurch gekennzeichnet, daß man ein Anhydrid nach einem der Ansprüche 1, 2, 3 oder 5, worin Ar wie in einem der Ansprüche 1, 2 oder 3 definiert ist, R₁ einen Rest tert.-Butoxycarbonyl bedeutet und R₂ und R₃ zusammen einen wie in einem der Ansprüche 1 oder 5 definierten gesättigten Heterocyclus mit 5 oder 6 Ringgliedern bilden, mit einem Derivat von Baccatin III oder von 10-Desacetyl-Baccatin III der allgemeinen Formel 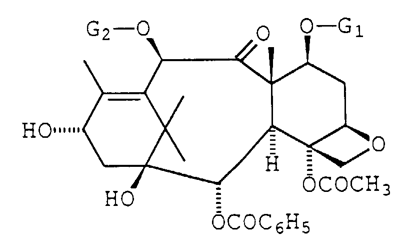 zur Reaktion bringt, in der G₁ eine Schutzgruppe für die Hydroxyfunktion darstellt und G₂ einen Acetylrest oder eine Schutzgruppe für die Hydroxyfunktion bedeutet, um ein Produkt der allgemeinen Formel 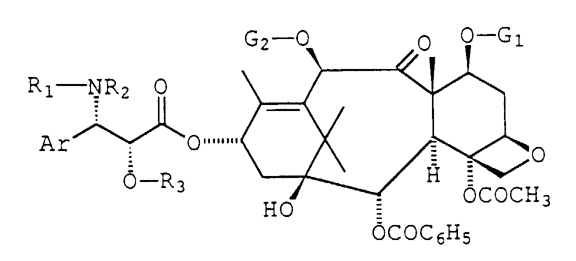 zu erhalten, worin R₁ einen Rest tert.-Butoxycarbonyl bedeutet, R₂ und R₃ zusammen einen gesättigten Heterocyclus mit 5 oder 6 Ringgliedern bilden und Ar wie in einem der Ansprüche 1, 2 oder 3 definiert ist, G₁ eine Schutzgruppe für die Hydroxyfunktion darstellt und G₂ einen Acetylrest oder eine Schutzgruppe für die Hydroxyfunktion bedeutet, die man im sauren Medium unter solchen Bedingungen behandelt, daß sie ohne Auswirkung auf die Schutzgruppen G₁ und G₂ sind, um ein Produkt der allgemeinen Formel 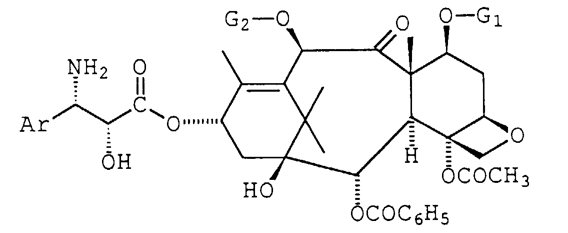 zu erhalten, in der Ar, G₁ und G₂ wie oben definiert sind, das man anschließend mit einer Verbindung behandelt, die es ermöglicht, bei der Aminfunktion einen Rest Benzoyl oder tert.-Butoxycarbonyl einzuführen, und man danach die Schutzgruppen G₁ und G₂ nach bekannten Methoden durch Wasserstoffatome ersetzt.

## Claims

1. New anhydrides of general formula: 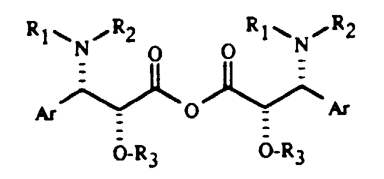 in which Ar represents an aryl radical, and, either R₁ represents a benzoyl or tert-butoxycarbonyl radical, R₂ represents a hydrogen atom and R₃ represents a protecting group of the hydroxyl functional group, or R₁ represents a tert-butoxycarbonyl radical and R₂ and R₃ together form a 5- or 6-membered saturated heterocycle.

2. New anhydrides according to claim 1, characterised in that Ar represents an optionally substituted phenyl or α- or β-naphthyl radical, the substituents being chosen from halogen atoms and alkyl, aryl, arylalkyl, alkoxy, alkylthio, aryloxy, arylthio, hydroxyl, mercapto, acylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals.

3. New anhydrides according to claim 1, characterised in that Ar represents a phenyl radical optionally substituted with a chlorine or fluorine atom, or with an alkyl radical containing 1 to 4 carbon atoms, an alkoxy radical containing 1 to 4 carbon atoms, a dialkylamino radical, each alkyl part of which contains 1 to 4 carbon atoms, an acylamino radical containing 1 to 4 carbon atoms or an alkoxycarbonylamino radical containing 1 to 4 carbon atoms.

4. New anhydrides according to one of claims 1, 2 or 3, characterised in that, when R₂ represents a hydrogen atom, R₃ represents a methoxymethyl, 1-ethoxyethyl, benzyloxymethyl, (β-trimethylsilylethoxy)methyl, tetrahydropyranyl, 2,2,2-trichloroethoxymethyl or 2,2,2-trichloroethoxycarbonyl radical.

5. New anhydrides according to one of claims 1, 2 or 3, characterised in that R₂ and R₃ together form an oxazolidine ring which is optionally gem-disubstituted in the 2-position.

6. Process for the preparation of an anhydride according to one of claims 1, 2, 3, 4 or 5, characterised in that a dehydrating agent is reacted with an acid of general formula: 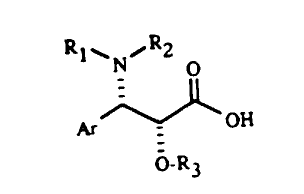 in which Ar, R₁, R₂ and R₃ are defined as in claims 1, 2, 3, 4 or 5.

7. Process according to claim 6, characterised in that the dehydrating agent is dicyclohexylcarbodiimide.

8. Process according to one of claims 6 or 7, characterised in that the reaction is carried out in an organic solvent chosen from halogenated aliphatic hydrocarbons and aromatic hydrocarbons.

9. Process according to one of claims 6, 7 or 8, characterised in that the reaction is carried out at a temperature of between 0 and 30°C.

10. Use of an anhydride according to one of claims 1, 2, 3 or 4, optionally prepared in situ, for the preparation of a product of general formula: 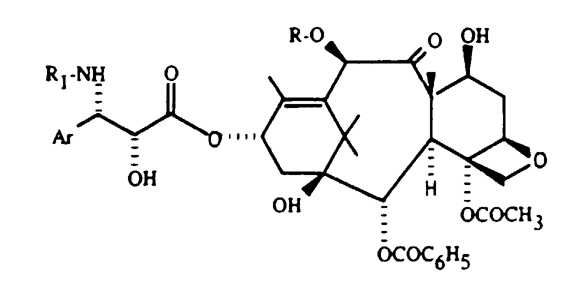 in which R represents a hydrogen atom or an acetyl radical, R₁ represents a benzoyl or tert-butoxycarbonyl radical, and Ar is defined as in one of claims 1, 2 or 3, characterised in that an anhydride according to one of claims 1, 2, 3 or 4, for which Ar is defined as in one of claims 1, 2 or 3, R₁ represents a benzoyl or tert-butoxycarbonyl radical, R₂ represents a hydrogen atom and R₃ represents a protecting group of the hydroxyl functional group as defined in claims 1 or 4, is reacted with a derivative of baccatin III or of 10-deacetylbaccatin III of general formula: 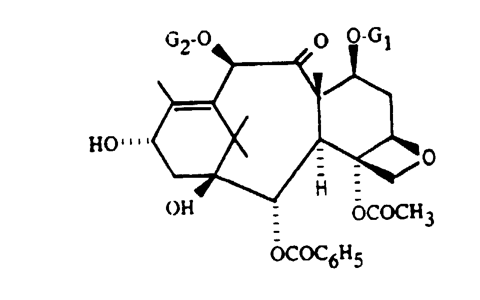 in which G₁ represents a protecting group of the hydroxyl functional group and G₂ represents an acetyl radical or a protecting group of the hydroxyl functional group, in order to obtain a product of general formula: 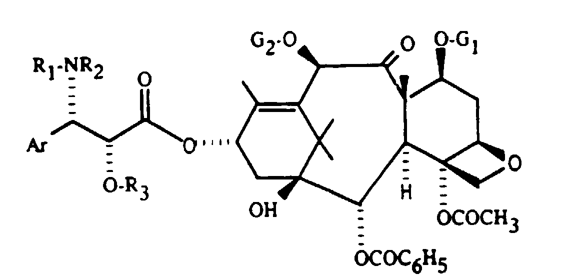 in which Ar, R₁, R₂, R₃, G₁ and G₂ are defined as above, and then the protecting groups R₃, G₁ and G₂ are replaced by a hydrogen atom by application of known methods.

11. Use of an anhydride according to one of claims 1, 2, 3 or 5, optionally prepared in situ, for the preparation of a product of general formula: 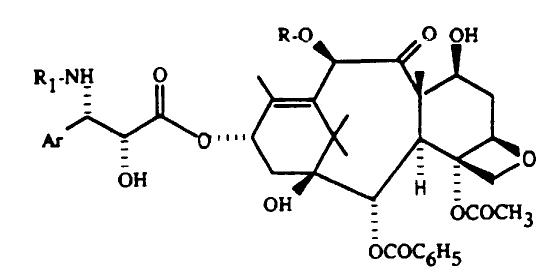 in which R represents a hydrogen atom or an acetyl radical, R₁ represents a tert-butoxycarbonyl radical, and Ar is defined as in one of claims 1, 2 or 3, characterised in that an anhydride according to one of claims 1, 2, 3 or 5, for which Ar is defined as in one of claims 1, 2 or 3, R₁ represents a tert-butoxycarbonyl radical and R₂ and R₃ together form a 5- or 6-membered saturated heterocycle as defined in either of claims 1 and 5, is reacted with a derivative of baccatin III or of 10-deacetylbaccatin III of general formula: 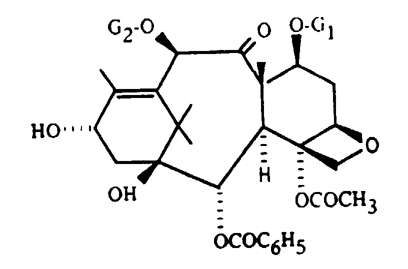 in which G₁ represents a protecting group of the hydroxyl functional group and G₂ represents an acetyl radical or a protecting group of the hydroxyl functional group, in order to obtain a product of general formula: 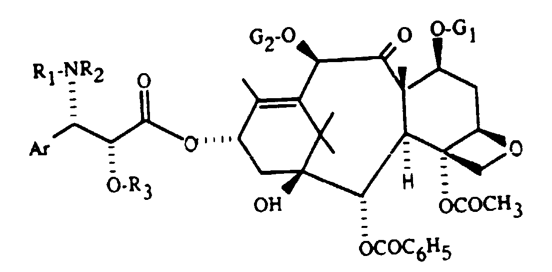 in which R₁ represents a tert-butoxycarbonyl radical, R₂ and R₃ together form a 5- or 6-membered saturated heterocycle and Ar is defined as in one of claims 1, 2 or 3, G₁ represents a protecting group of the hydroxyl functional group and G₂ represents an acetyl radical or a protecting group of the hydroxyl functional group, which product is treated in acidic medium, under conditions which do not affect the protecting groups G₁ and G₂, in order to obtain a product of general formula: 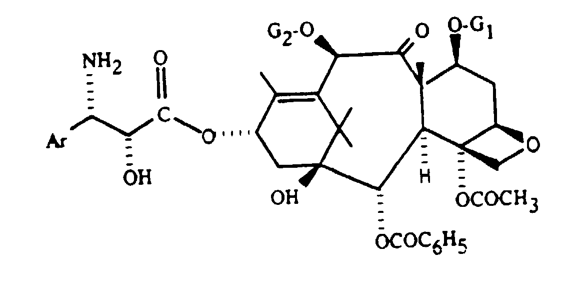 in which Ar, G₁ and G₂ are defined as above, which product is treated with a compound which makes it possible to introduce a benzoyl or tert-butoxycarbonyl radical onto the amino functional group, and the protecting groups G₁ and G₂ are then replaced by a hydrogen atom according to known methods.
